## Europäisches Patentamt
## European Patent Office
## Office européen des brevets

(11) Veröffentlichungsnummer: **0 008 758**
**B1**

(12) **EUROPÄISCHE PATENTSCHRIFT**

(45) Veröffentlichungstag der Patentschrift:
**21.11.84**

(21) Anmeldenummer: **79103144.6**

(22) Anmeldetag: **27.08.79**

(51) Int. Cl.³: **A 61 B 17/18,** A 61 F 5/04, A 61 F 1/00

(54) Verfahren zur Herstellung eines Knochenmarknagels.

(30) Priorität: **04.09.78 AT 6368/78**

(43) Veröffentlichungstag der Anmeldung:
**19.03.80 Patentblatt 80/6**

(45) Bekanntmachung des Hinweises auf die Patenterteilung:
**21.11.84 Patentblatt 84/47**

(84) Benannte Vertragsstaaten:
**BE CH DE FR GB IT NL SE**

(56) Entgegenhaltungen:
**AT - B - 246 326
CH - A - 497 891
DE - A - 2 361 933
DE - A - 2 609 723
DE - A - 2 701 279
DE - C - 913 228
FR - A - 2 288 506
FR - A - 2 342 710
US - A - 3 605 123
US - A - 4 040 129
US - A - 4 103 683**

**Dr. OTTO-ALBRECHT NEUMÜLLER "RÖMPPS CHEMIE-LEXIKON", 7. Auflage, Franckh'sche Verlagshandlung, Stuttgart, 1977; Seite 3463**

(73) Patentinhaber: **METALLWERK PLANSEE GESELLSCHAFT MBH, Siebenbürgerstrasse 23, D-8923 LECHBRUCK (DE)**
Patentinhaber: **Otte, Wolf-Dieter, Am Model 14, D-8217 Volkach (DE)**

(72) Erfinder: **Otte, Heinz, Dr. med., Am Model 14, D-8712 Volkach (DE)**
Erfinder: **Otte, Wolf-Dieter, Am Model 14, D-8712 Volkach (DE)**
Erfinder: **Schider, Siegfried, Dr., Mittenwaldbahnstrasse 12, A-6600 Reutte-Breitenwang (AT)**
Erfinder: **Wiesner, Otto, Runzfeld 3, A-6600 Reutte-Mühl (AT)**

(74) Vertreter: **Lohnert, Wolfgang, Dr., Metallwerk Plansee AG, A-6600 Reutte, Tirol (AT)**

## Beschreibung

Die Erfindung betrifft ein Verfahren zur Herstellung eines Knochenmarknagels zur Frakturfixierung, der über seine annähernd volle Länge ein äußeres Profil, bestehend aus Kreisabschnitten im Wechsel mit kreissegmentartig eingebuchteten Zonen zur verdrehfesten Fixierung des Knockenmarknagels im Markkanal sowie eine vorzugsweise kreisförmige Schneide an einem Ende aufweist.

Ein Knochennagel mit dieser Formgebung ist in der FR-A1-2 288 506 beschrieben. Er ist als massiver Rundstab mit im Profil halbkreisförmigen Längsnuten und einer schneidenförmigen Spitze an einem Ende ausgeführt.

Die DE-C-913 228 beschreibt längsgeschlitzte Knochenmarknägel der verschiedensten Querschnitte u. a. auch in etwa des oben beschriebenen Profils. Die Festigkeit derartiger Nägel wurde teilweise durch »Prägen« einzelner Wandlängsstreifen erhöht. Diese haben infolge des »Prägens« eine kleinere Wanddicke als die übrigen Bereiche des Nagels.

Ein in der FR-A1-2 342 710 beschriebener Knochennagel zur Halterung einer Knochenplatte weist Rohrform auf und besitzt an der äußeren Oberfläche eingefräste Längsnuten mit kreissegmentartigem Profil.

Aus der DE-A-2 361 933 ist ein rohrförmiger Knochenmarknagel bekannt, der auf seiner äußeren Oberfläche im Unterschied zu den vorstehend beschriebenen Nägeln Längserhebungen aufweist, die in eine Schneidenfläche an einem Rohrende auslaufen. Diese Profilform hat den Nachteil, daß beim Einschlagen erhebliche Mengen an im Markraum verbleibendem Knochenmaterial ausgeschnitten werden und daß im Bereich der spitzen Längserhebungen ein ungünstig hoher Druck vom Knochenmarknagel auf das Knochenmaterial ausgeübt wird, was die Gefahr der Knochensplitterung und Rißbildung zur Folge hat.

Werkstoffe für Knochenmarknägel sollten eine hohe Zug- und Biegebruchfestigkeit, eine ausreichende Dehnung und eine möglichst hohe Korrosionsfestigkeit oder Gewebeverträglichkeit aufweisen. Man verwendet bis heute in der Regel Chrom-, Molybdän- und Nickelstähle. Obwohl die genannten Materialien zu den nichtrostenden Stählen zu zählen sind, treten bei deren Verwendung im lebenden Organismus unerwünschte chemische Korrosionsreaktionen, beispielsweise Spannungsrißkorrosion und Lochfraß, auf. Dadurch werden die Implantate in ihrer Funktion beeinträchtigt oder unbrauchbar. Zugleich wird durch die Reaktionen zwischen Implantatwerkstoff und organischem Gewebe das Gewebe selbst geschädigt und eine Heilung der Knochenfraktur erschwert oder unmöglich gemacht. Derartige Erscheinungen sind unter dem Begriff Metallose bekannt.

Es hat daher nicht an Versuchen gefehlt, von den genannten Stahlsorten als Werkstoff für Knochenmarknägel wegzukommen und an deren Stelle beispielsweise Legierungen auf der Basis von Kobalt oder Titan zu verwenden. Es wurde auch vorgeschlagen, die genannten Werkstoffe oberflächlich durch Oxidieren, Nitrieren oder Karburieren zu veredeln oder Keramikschichten aufzutragen.

Im Zusammenhang mit der Diskussion der Metallose-Erscheinungen wurde in der Implantat Chirurgie in der Vergangenheit der Werkstoff Tantal wegen seiner hohen Gewebeverträglichkeit gelegentlich erwähnt. Die Lehrmeinung besagt aber, daß Tantal wegen seiner geringen Festigkeitseigenschaften nur dort verwendet werden kann, wo die Duktilität, nicht aber die Festigkeit des Materials im Vordergrund steht, zum Beispiel bei feinen Drähten und Netzen. Außerdem werden das hohe spezifische Gewicht und der hohe Preis als Grund dafür genannt, das Tantal nicht in breiterem Umfang als Implantatwerkstoff verwendet werden kann.

Es ist üblich, die Festigkeitseigenschaften metallischer Werkstoffe durch Warm- und Kaltverformung, wie Walzen und Ziehen, zu steigern. Es ist aber so bekannt, daß gerade hochschmelzende Metalle, wie Niob und Tantal, nur vergleichsweise beschränkt verformbar sind, denn mit einer Steigerung der Festigkeit durch Verformung und/oder Legierungszusätze geht in der Regel gleichzeitig eine starke Versprödung einher.

In der US-A-3 605 123 sind ganz allgemein Prothesenteile, wie Platten, Nägel, Stifte und Schrauben, aufgeführt, die aus verschiedenartigen »hochfesten Materialien«, unter anderem aus Tantal, gefertigt sein können. In der Veröffentlichung ist offengelassen, ob Tantal in reiner Form oder nur als Legierungsbestandteil Verwendung findet. Knochenmarknägel, an die besonders hohe mechanische Anforderungen gestellt werden, sind in ihr nicht erwähnt.

Auch in »Römmps Chemie Lexikon«, 1977, ist auf Seite 3464 erwähnt, daß Tantal in der Chirurgie als Werkstoff für Knochennägel, Knochenersatzstücke, Klammern und Kieferschrauben verwendet wird. Aber auch hier sind mechanisch besonders hoch beanspruchte Knochenmarknägel nicht erwähnt.

Die US-A-4 040 129 beschreibt als Implantat-Werkstoff Metallegierungen mit maximal 30 Gew.-% Tantal bzw. Niob und stützt damit die allgemeine Auffassung, daß aus Festigkeitsgründen speziell für mechanisch hochbeanspruchte Implantatteile, zum Beispiel Knochenmarknägel, kein reines oder schwach legiertes Tantal oder Niob in Frage kommt.

Aufgabe der Erfindung ist es, ein Verfahren zur Herstellung von Knochenmarknägeln mit dem eingangs beschriebenen äußeren Profil zu schaffen, daß die Verwendung der gewebeverträglichen Metalle Tantal und Niob ermöglicht und mit dem die erforderliche Festigkeit und Elastizität des Nagels, verbunden mit einer gewissen plastischen Verformbarkeit, mit vergleichsweise geringem Materialeinsatz erzielt werden.

Diese Aufgabe wird erfindungsgemäß dadurch gelöst, daß ein rohrförmiger Rohling aus Tantal oder aus einer mehr als 50 Gew.-% Tantal enthaltenden Legierung oder aus Niob oder aus einer mehr als 50 Gew.-% Niob enthaltenden Legierung durch spanlose Formgebung derart warm- und/oder kaltverformt wird, daß ein Knochenmarknagel konstanter Wanddicke mit mindestens 5 Kreisabschnitten gebildet wird, dessen Kreisabschnitte scharfkantig an die kreissegmentartig eingebuchteten Zonen angrenzen.

Durch das spezielle, eine einheitliche Wanddicke aufweisende Profil des erfindungsgemäß hergestellten Knochenmarknagels erhält der Werkstoff an jeder Stelle annähernd die gleiche hohe Verfestigung. Es treten somit keine Bereiche unterschiedlicher Verformung und somit unterschiedlicher Festigkeit auf. Dadurch sind die inneren Spannungen des Materials gering. Im Gegensatz zu Stahlnägeln zeigt ein Niob- oder Tantal-Nagel selbst beim Auftreten von Mikrorissen im Werkstoff keine Anfälligkeit für die Spannungsrißkorrosion. Die Verformung des Werkstoffes läßt sich in allen Bereichen gleichmäßig bis zu einer Grenzfestigkeit durchführen, bei der die mit der Festigkeit ebenfalls ansteigende Versprödung des Niob- oder Tantal-Werkstoffes gerade noch tolerierbar ist.

Erst seit kurzer Zeit vorliegende Messungen haben ergeben, daß die Reaktionsträgheit von Knochenmarknägeln aus Tantal, aber auch aus Niob um einige Zehnerpotenzen größer ist als die bekannter Knochenmarknägel.

Für derartige Nägel üblicherweise eingesetzte Chrom-Molybdän-Nickelstähle weisen zwar Zugfestigkeiten bis zu 1000 N/mm² und zugleich Dehnungen von 30—40% auf, Werte, welche bei Nägeln aus Niob und Tantal sowie deren Legierungen gemäß der Erfindung nicht gleichzeitig erreichbar sind.
Gleichwohl liegen die Festigkeitswerte von erfindungsgemäßen Knochemarknägeln innerhalb der Bandbreite, welche unter der ASTM-Norm F 383 für auf dem Markt befindliche Knochenmarknägel angegeben ist. Daneben ist das plastische Durchbiegungsverhalten eines erfindungsgemäß hergestellten Tantal-Nagels aber weit größer als das eines vergleichbaren Stahl-Nagels.

Diese vorteilhafte Eigenschaft des erfindungsgemäßen Knochenmarknagels erlaubt dem Chirurgen gegebenenfalls noch während der Operation Formkorrekturen am Nagel vorzunehmen, um diesen der Form des Knochenkanals möglichst exakt anzupassen.

Durch die spezielle Formgebung des erfindungsgemäß hergestellten Knochenmarknagels läßt sich bei noch ausreichenden Festigkeitseigenschaften der Materialaufwand so klein halten, daß im Falle von Niob als Nagelwerkstoff sein Gewicht dem herkömmlicher Knochenmarknägel aus Stahl entspricht. Doch auch bei Verwendung von Tantal und der anderen erfindungsgemäßen Werkstoffe wird das Nagelgewicht vom medizinischen Standpunkt aus nicht als nachteilig empfunden.

Die Profilform eines erfindungsgemäß hergestellten Knochemarknagels ergibt infolge der scharfkantigen Grenzen zwischen einzelnen Zonen eine sehr gute Verdrehsicherheit im Knochen, ohne daß beispielsweise die Nachteile eines Nagels mit nach außen spitz zulaufenden Erhebungen auftreten.

Bei einem bevorzugten Herstellungsverfahren wird die Oberfläche des Knochenmarknagels in bekannter Weise anodisch oxidiert.

Bei einem weiteren bevorzugten Verfahren zur Herstellung von Knochenmarknägeln wird ein Sinterkörper aus Niob oder Tantal oder aus einer mehr als 50 Gew.-% Niob oder Tantal enthaltenden Legierung zu einem Blech ausgewalzt und dieses in geschlitzte Rohrform gebracht. Anschließend wird der rohrförmige Körper zu einem geschlossenen Rohr verschweißt. Es erfolgt dann die endgültige Profilgebung, insbesondere die Einbringung der segmentartig eingebuchteten Zonen, durch Rollen, Schmieden oder Pressen.

Anschließend wird der Knochenmarknagel allseitig entgratet und die Mantelfläche poliert.

Nach einem weiteren bevorzugten Herstellungsverfahren wird die Vorform des Knochenmarknagels durch Strangpressen eines rohrförmigen Sinterkörpers aus Niob oder Tantal erzeugt. Anschließend wird das Rohr durch Ziehen und/oder Schmieden auf kleinere Durchmesser gebracht. Der weitere Herstellungsablauf entspricht dem des vorausgegangenen Beispieles.

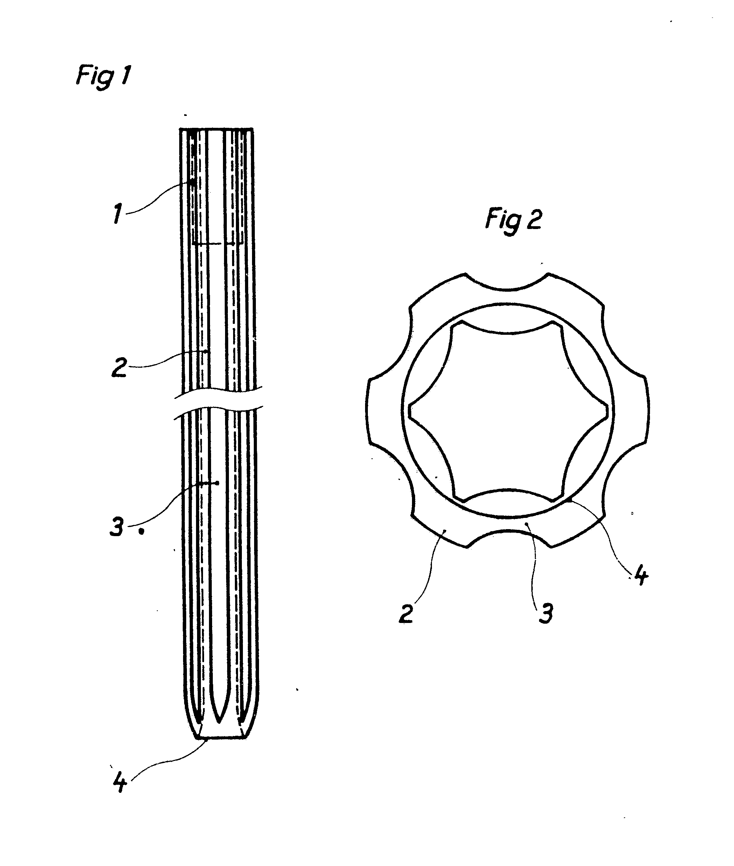

Die Fig. 1 und 2 zeigen eine bevorzugte Formgebung eines erfindungsgemäß hergestellten Knochenmarknagels.

Fig. 1 zeigt den Knochenmarknagel in Seitenansicht.

Fig. 2 zeigt die Draufsicht auf das die Schneide enthaltende Ende des Knochenmarknagels gemäß Fig. 1.

In Fig. 1 ist zu erkennen, daß der Knochenmarknagel sich nach unten zur Schneide 4 hin verjüngt. Die Figur zeigt über die gesamte Länge des Nagels etwa gleichbleibende Längsnuten in der äußeren Mantelfläche. Dabei grenzen Kreisabschnitte 2 im Wechsel an kreissegmentartig eingebuchtete Zonen 3. Der dargestellte Knochenmarknagel weist an dem der Schneide gegenüberliegenden Ende ein Innengewinde 1 auf.

Die Draufsicht auf die Schmalseite gemäß Fig. 2 läßt das Profil des Knochenmarknagels erkennen. Auffallend ist, daß die innere Begrenzung der äußeren Begrenzung des Knochenmarknagels so angepaßt ist, daß die Wanddicke über das gesamte Profil etwa gleich ist. Diese Profilform ist gewählt, damit der Knochenmarknagel eine große Festigkeit und Steifigkeit bei möglichst geringer Wanddicke und damit möglichst geringem Materialeinsatz aufweist. Es ist ferner zu entnehmen, daß der Knochenmarknagel im Profil sechs Kreisabschnitte 2 aufweist, die jeweils im Wechsel scharfkantig an kreissegmentartig eingebuchtete Zonen 3 angrenzen.

## Patentansprüche

1. Verfahren zur Herstellung eines Knochenmarknagels zur Frakturfixierung, der über seine annähernd volle Länge ein äußeres Profil, bestehend aus Kreisabschnitten (2) im Wechsel mit kreissegmentartig eingebuchteten Zonen (3) zur verdrehfesten Fixierung des Knochenmarknagels im Markkanal sowie eine vorzugsweise kreisförmige Schneide an einem Ende aufweist, dadurch gekennzeichnet, daß ein rohrförmiger Rohling aus Tantal oder aus einer mehr als 50 Gew.-% Tantal enthaltenden Legierung oder aus Niob oder aus einer mehr als 50 Gew.-% Niob enthaltenden Legierung durch spanlose Formgebung derart warm- und/oder kaltverformt wird, daß ein Knochenmarknagel konstanter Wanddicke mit mindestens fünf Kreisabschnitten (2) gebildet wird, dessen Kreisabschnitte scharfkantig an die kreissegmentartig eingebuchteten Zonen (3) angrenzen.

2. Verfahren zur Herstellung eines Knochenmarknagels nach Anspruch 1, dadurch gekennzeichnet, daß er in bekannter Weise auf seiner Oberfläche anodisch oxidiert wird.

3. Verfahren zur Herstellung eines Knochenmarknagels nach Anspruch 1 oder 2, dadurch gekennzeichnet, daß ein Sinterkörper aus Niob oder Tantal oder aus einer mehr als 50 Gew.-% Niob oder Tantal enthaltenden Legierung zu einem Blech ausgewalzt wird, das Blech in geschlitzte Rohrform gebracht und zu einem geschlossenen Rohr verschweißt wird und daß abschließend durch Rollen, Schmieden oder Pressen die kreisförmig eingebuchteten Zonen eingebracht werden.

4. Verfahren zur Herstellung eines Knochenmarknagels nach Anspruch 1 oder 2, dadurch gekennzeichnet, daß ein rohrförmiger Sinterkörper aus Niob oder Tantal oder aus einer mehr als 50 Gew.-% Niob oder Tantal enthaltenden Legierung durch Strangpressen in eine Vorform gebracht wird, diese Vorform durch Schmieden oder Ziehen unter neuerlicher Verminderung des Durchmessers und der Wanddicke auf den endgültigen Durchmesser gebracht wird und schließlich durch Rollen, Schmieden oder Pressen die kreisförmig eingebuchteten Zonen eingebracht werden.

## Claims

1. Method for the production of a medullary nail for fracture fixation, having along its nearly entire length an outer shape comprising segments being annular in shape (2) and altering with segments being of concave annular shape (3), resulting in a nail rotation stable fixed in the bone marrow and having a preferably annular cutter at the one end thereof, characterized in, that a tubular blank of tantalum or an alloy comprising more than 50% by weight tantalum or of niobium or an alloy comprising more than 50% by weight niobium is shaped by chipless forming in its cold or hot aggregate to the end that a nail of constant wall shickness with at least five segments of annular shape is performed, and that the segments of annular shape being bounded by sharp edges to said segments of concave annular shape.

2. Method for the production of a medullary nail according to claim 1, wherein the nail is oxidized on its surface in a known manner.

3. Method for the production of a medullary nail according to claim 1 or 2, wherein a sinter blank of niobium or tantalum or an alloy comprising more than 50% by weight niobium or tantalum will be rolled into a sheet, the sheet will be brought into slotted tubular from and then welded to a tube, and where in a final step the segments of concave annular shape are formed by rolling with rollers, by forging or pressing.

4. Method for the production of a medullary nail according to claim 1 through 3, wherein a tubular sinter blank of niobium or tantalum or of an alloy comprising more than 50% by weight niobium or tantalum is preshaped by extruding, the preshaped nail is brought to its final diameter by further reduction of its diameter and wall thickness in a swaging or drawing process and where in a final step the segments of concave annular shape are formed by rolling with rollers, by forging or pressing.

## Revendications

1. Méthode pour la production d'un clou médullaire pour fixer des fractures, ce clou médullaire a presque sur toute sa longueur un profil extérieur, consistant de segments de cercle (2) en saillie alternant avec des zones creusées en form de segments de cercle (3) pour la fixation resistante à la torsion du clou medullaire dans le canal médullaire; le clou a favorablement aussi une lame de préférance curculaire à une extrémité, caractérisé en ce qu'un lingot tubulaire en tantale ou en un alliage contenant plus de 50% en poids de tantale ou en niobium ou en un alliage contenant plus de 50% en poids du niobium est deformé à chaud et/ou à froid par déformation sans enlèvement de copeaux de facon qu'un cluo médullaire d'une épaisseur de paroi constante est formé avec au moins 5 segments de cercle (2), les segments de cercle duquel touchent les zones creusées en form de segments de cercle (3) avec des arêtes vives.

2. Méthode pour la production d'un clou médullaire suivant la revendication 1, caractérisé en ce qu'il est oxydé anodiquement sur sa surface de facon connue.

3. Méthode pour la production d'un clou médullaire suivant l'une de revendications 1 ou 2, caractérisé en ce qu'un comprimé fritté de nioubium ou tantale ou d'une alliage contenant plus de 50% en poids de niobium ou tantale est la- niné dans la forme d'une plaque, que la plaque est formée comme une tube fendue et est soudée en forme d'une tube fermée et qu'enfin les zones creusées en form de cercle sont placées par roulage, forgeage ou par pressage.

4. Méthode pour la production d'un clou médullaire suivant l'une de revendications 1 ou 2, caractérisé en ce qu'un comprimé fritté tubulaire de niobium ou tantale ou d'un alliage contenant de plus de 50% en poids de niobium ou tantale est donnée une préforme par extrusion, que cette préforme obtient le diamètre définitiv par forgeage ou étirage en diminuant le diamètre et l'épaisseur de paroi à nouveau et qu'enfin les zones creusées en form de segments de cercle sont placées par roulage, forgeage ou pressage.

**Fig 1**

**Fig 2**